# EUROPEAN PATENT APPLICATION

(11) **EP 3 835 407 A1**
(43) Date of publication of application: **16.06.2021**
(21) Application number: 19215389.8
(22) Date of filing: 11.12.2019
(51) Int. Cl.: C12M 1/12, C12M 1/00, C12M 1/34

(54) **TUBULAR BIOMANUFACTURING ASSEMBLY**

(71) Applicant: Sartorius Stedim Biotech GmbH, 37079 Göttingen (DE)
(72) Inventor: Prediger, Andreas, 37079 Göttingen (DE); Scholz, Jochen, 37079 Göttingen (DE); Grimm, Christian, 37079 Göttingen (DE); Fortuna, Ana-Raquel, 37079 Göttingen (DE); Zijlstra, Gerben, 37079 Göttingen (DE); Höhse, Marek, 37079 Göttingen (DE); Bode, Matthias, 37079 Göttingen (DE)
(74) Representative: Prinz & Partner mbB

(57) **Abstract**

A biomanufacturing assembly comprises a continuous elongated fluid channel (10) for hosting a biological reaction occurring in a fluid medium along a flow path of the fluid channel (10). The biomanufacturing assembly further comprises at least one monitoring component (16) enabling monitoring of the biological reaction and/or at least one interaction component (18) for actively influencing the biological reaction. The monitoring component (16) and/or the interaction component (18) is/are integrated within the continuous elongated fluid channel (10).

## Description

The invention relates to a tubular biomanufacturing assembly, in particular to an assembly comprising a continuous elongated fluid channel for hosting a biological reaction occurring in a fluid medium along a flow path of the fluid channel.

Tubular flow reactors (TFR), such as plug flow reactors, are well-established in the chemical industry. In general, the flow through such a reactor is continuous, usually at steady state, and configured so that conversion of the chemicals and other dependent variables are functions of axial position within the reactor rather than of time. In an ideal plug flow reactor, the medium with the reactants flows as if it was an array of solid plugs or pistons, and reaction time is the same for all flowing material at any given tube cross section. Tubular reactors provide initially high driving forces, which diminish as the reaction progresses down the tube.

Compared to continuous stirred tank reactor (CSTR) systems, the main characteristic of tubular flow reactor systems is the continuous gradient of the process maturity along the flow path. Possible advantages of tubular flow reactor systems include simple maintenance (especially since there are no internal mechanical means in the reaction system for agitating or stirring the medium), a larger surface to volume ratio (allowing a more efficient exchange of e.g. gas or nutritient), and a smaller variance of products as they generally have narrower residence time distributions than comparable continuous stirred tank reactor systems. This is because due to the flow regime there is no back-mixing, so e.g. in case of an induced viral infection it is ensured that the main source vessel will not be spoiled.

When tubular flow reactors are to be used for biomanufacturing, there is a trend towards disposable (single-use) systems due to their specific advantages, such as reduced contamination risk, time and cost savings as well as ease of use, especially as complicated cleaning of the bioreactor becomes obsolete.

However, hitherto known tubular flow reactor systems used for biomanufacturing lack the ability to monitor and control bioprocesses. It is therefore an object of the invention to overcome this drawback.

The above problem is solved by a tubular biomanufacturing assembly according to claim 1. Advantageous and expedient embodiments of the invention are apparent from the dependent claims.

The invention provides a tubular biomanufacturing assembly consisting of single-use components. The biomanufacturing assembly according to the invention comprises a continuous elongated fluid channel for hosting a biological reaction occurring in a fluid medium along a flow path of the fluid channel. The tubular biomanufacturing assembly further comprises at least one monitoring component enabling monitoring of the biological reaction and/or at least one interaction component for actively influencing the biological reaction. The monitoring component and/or the interaction component is/are integrated within the continuous elongated fluid channel.

The term "medium" is to be understood as containing certain reactants fed into the fluid channel at one end, e.g. viruses from a virus stock and cells provided by a chemostat. The resulting product of the biological reaction is withdrawn at another end of the fluid channel. "Actively influencing" is to be understood as an active interaction with the medium, such as changing or modifying the composition of the medium, in contrast to a passive influence like a given, invariable course of the flow path itself or of its cross section.

The invention is based on the finding that the biological reaction occurring in a tubular flow reactor used for biomanufacturing can be effectively controlled when one or more parameters of the medium can be monitored or actively influenced while the bioprocess is running. With the monitoring component and/or the interaction component integrated within the fluid channel of the biomanufacturing assembly it is possible to monitor and/or to actively influence the biological reaction without the need of taking samples, providing bypass lines or interposing any analytical devices in the flow path etc.

According to the invention, the whole biomanufacturing assembly consists of single-use components. It is much easier to use and then dispose the biomanufacturing assembly as a whole afterwards. Otherwise it would be necessary to provide sterile connections between single-use and reusable components and to separate them before disposing the single-use components.

The monitoring component of the tubular biomanufacturing assembly enabling monitoring of the biological reaction in the fluid channel preferably includes at least one sensor. The sensor output can be provided to a control unit for evaluation and adapting process control parameters accordingly, if required. Preferably, these steps are performed automatically.

The following types of sensors are especially useful for monitoring the medium and the biological reaction: pH sensor (e.g. optochemical or electrochemical sensor); dissolved oxygen sensor; dissolved carbon dioxide sensor; microscope; fiber Bragg sensor; conductivity sensor; capacitance sensor; refractometer; flow sensor; pressure sensor; optical sensor (e.g. UV, Raman, NIR, fluorescence); exhaust gas analyzer (e.g. IR spectroscope); image sensor (e.g. hyperspectral imager; holographic phase imager).

The interaction component of the tubular biomanufacturing assembly for actively influencing the biological reaction preferably includes at least one structure that is adapted to exchange chemical substances with the medium in the fluid channel. By exchanging chemical substances the composition of the medium can be changed or modified in order to control the biological reaction in a desired manner. It is to be noted that exchanging chemical substances shall include transfer of gas into the fluid channel, either directly or in the liquid phase via a membrane (e.g. transfer of dissolved oxygen).

According to a first alternative, the structure can be adapted to feed at least one substrate and/or at least one inducer to the medium. Examples for a substrate to be fed are oxygen, carbon and nitrogen. Accordingly, the structure may include corresponding sources of the desired substrates. Examples for inducers to be fed are specific sugars, enzymes or viruses. Another example is isopropyl β-D-1-thiogalactopyranoside (IPTG), which can be used to induce Escherichia coli (E. coli) to start production of a protein.

According to a second alternative, the structure can be adapted to withdraw dissolved carbon dioxide and/or at least one metabolite and/or at least one product from the medium in order to influence the biological reaction in the fluid channel.

Of course, it is generally possible to have both "feeding" structures and "withdrawing" structures at the same location or at different locations in the fluid channel.

Moreover, it is even possible that the structure of the interaction component is adapted to draw at least one sample from the medium for offline examination. The sample can be a cell-free or a cell-containing sample.

The structure of the interaction component may include a permeable or a semi-permeable membrane, like a membrane used for dialysis, and/or a ceramic material to provide the desired filter or separation characteristics.

According to an alternative embodiment, the structure of the interaction component includes at least one hollow fiber.

The elongated fluid channel of the tubular biomanufacturing assembly can be part of a stand-alone tubing, e.g. made of silicone, or a reinforced tubing, or part of a rigid piping.

According to a special embodiment of the invention, the fluid channel is fixed in a cassette or cartridge (hereinafter commonly referred to as cassette). This design allows easy handling of the biomanufacturing assembly and potential incorporation into a greater process assembly. Further, the cassette may be adapted to accommodate further useful components and/or further fluid channels which can be fixed therein.

It is also possible that the biomanufacturing assembly includes any combination of (i) a stand-alone tubing, (ii) a rigid piping, and (iii) a cassette. Accordingly, any of these assembly structures may contain a fluid channel as described above, or one such fluid channel may extend through more than one of these assembly structures.

To induce optimal mixing of the fluid medium in the fluid channel, at least one mixing device can be integrated within the fluid channel. The mixing device may include baffles and/or a static mixer, for example.

Mixing of the medium can also be achieved, or at least supported, by the choice of the materials from which the fluid channel is built. In particular, different sections of the inner wall of the fluid channel can be made of materials with different surface properties. For example, variation in surface friction causes different flow velocities of the medium in the peripheral region of the medium, resulting in a turbulent flow.

A further measure to achieve or support mixing of the medium is the design of the course of the fluid channel. In particular, the fluid channel can include at least one meandering and/or at least one spiral section. Especially spiral sections with varying lead angles (inclination) and/or polar slope angles can be used to achieve a better radial and/or axial mixing behavior.

The biological reaction can also be influenced by varying temperatures, i.e. by heating or cooling different sections of the fluid channel differently. Especially in a tubular biomanufacturing assembly with a fluid channel fixed in a cassette, the fluid channel can be guided through different temperature zones.

It is also possible to provide more complex flow paths in the biomanufacturing assembly according to the invention. To this end, the fluid channel may be provided with several individual output and/or input branches and/or openings at different locations with respect to the overall length of the fluid channel. This design aspect offers a great variety of operation modes. For example, with different output branches the point of harvest can be selected as desired. Additional input branches enable addition of buffers or other fluids at desired locations. Moreover, input and an output branches can be combined to provide bypass paths. Such a bypass path allows circumvention of a possibly blocked or contaminated passage of the main path. Further, a bioreactor, especially a stirred tank reactor (STR), can be combined with the tubular biomanufacturing assembly via an additional input or output branch. It is generally possible (i) to use the bioreactor in a preceding step, letting the medium exit the bioreactor and enter the fluid channel of the tubular biomanufacturing assembly via an input branch, or (ii) to use the bioreactor in a subsequent step, letting the medium exit the tubular biomanufacturing assembly via an output branch and enter the bioreactor, or (iii) to use the bioreactor in an intermediate step by connecting an output branch of the tubular biomanufacturing assembly with an inlet of the bioreactor and an input branch of the tubular biomanufacturing assembly with an outlet of the bioreactor.

Further features and advantages of the invention will become apparent from the following description and from the accompanying drawings to which reference is made. In the drawings:
- Figure 1 shows a sectional view of a first embodiment of a tubular biomanufacturing assembly according to the invention;
- Figure 2 shows a sectional view of a second embodiment of a tubular biomanufacturing assembly according to the invention; and
- Figure 3 shows a sectional view of a third embodiment of a tubular biomanufacturing assembly according to the invention.

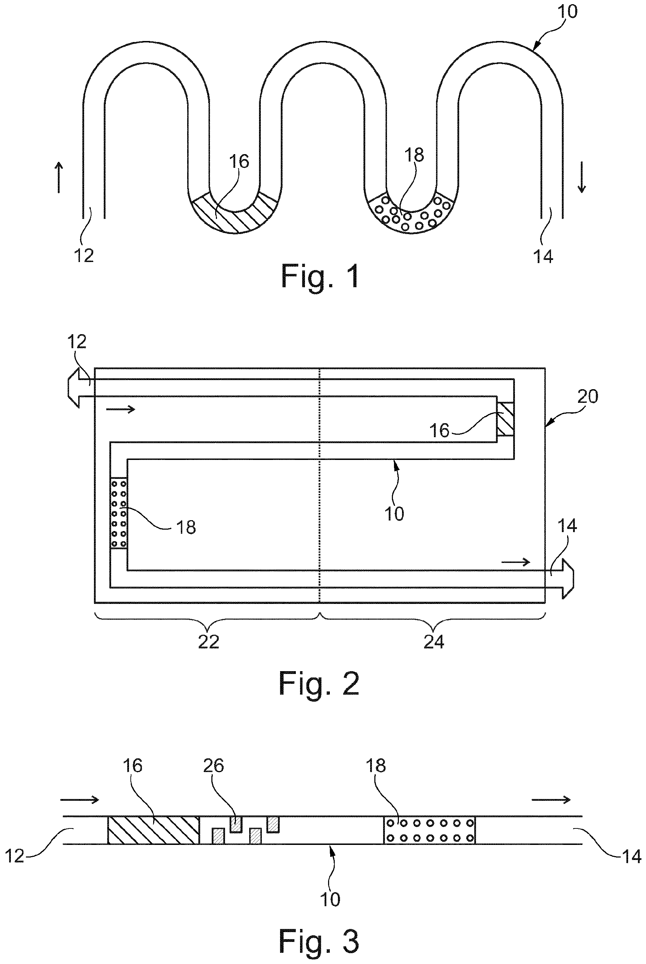

Figures 1, 2 and 3 depict different embodiments of a disposable tubular biomanufacturing assembly comprising a continuous (i.e. non-interrupted) elongated fluid channel 10. The fluid channel 10 may be a straight channel, or it may include significantly curved sections, like helical, spiral or meandering sections.

Reactants are continuously fed into the fluid channel 10 at a first end 12. For example, for a continuous virus production, cells produced in continuous mode in a chemostat and viruses from a virus stock or a transfection solution are continuously fed into the fluid channel 10. The medium containing the reactants is urged (e.g. by means of a pump, vacuum, compressed or pressurized air, or gravity) to flow through the fluid channel 10 in a flow direction (indicated by the arrows) to a second end 14 of the fluid channel 10. Along the fluid channel 10, a biological reaction occurs in the medium, e.g. infection/transfection of the cells, and the product of the biological reaction is continuously harvested at the second end 14 of the fluid channel 10. In some applications, the product of the biological reaction or parts of it are used to feed or inoculate the inlet of another tubular biomanufacturing assembly.

At least one monitoring component 16 and/or at least one interaction component 18 are accommodated in the fluid channel 10. More specifically, the components 16, 18, are not interposed between and connected to separate adjacent tubes, but rather integrated within the continuous fluid channel 10.

The monitoring component 16 enables monitoring of the biological reaction and is connected to a control unit (not shown). The output of the monitoring unit is evaluated, and the control unit may adapt certain process control parameters (e.g. feed rates) based on the evaluation.

In particular, the monitoring component 16 includes at least one sensor for detecting or measuring parameters of the medium which are characteristic for the biological reaction and/or the medium in the fluid channel 10. Depending on the reactants, the biological reaction and other circumstances, sensors of the following types may be useful: pH sensor (e.g. an optochemical or electrochemical pH sensor); dissolved oxygen sensor; dissolved carbon dioxide sensor; microscope; fiber Bragg sensor; conductivity sensor; capacitance sensor; refractometer; flow sensor; pressure sensor; optical sensor (e.g. UV, Raman, NIR, fluorescence); exhaust gas analyzer (e.g. IR spectroscope); image sensor (e.g. hyperspectral imager; holographic phase imager).

The interaction component 18 is provided to actively influence the biological reaction. In particular, the interaction component 18 includes at least one structure adapted to exchange chemical substances with the medium in the fluid channel 10. "Exchanging" means either feeding one or more substances to the medium from or with one or more sources provided in the interaction component 18, or withdrawing one or more substances from the medium. It is even possible to have a structure accomplishing both, i.e. feeding and withdrawing certain substances at the same time.

In order to provide the feeding and/or withdrawing functionality, the structure of the interaction component 18 includes a permeable or a semi-permeable membrane and/or a ceramic material, or the structure includes at least one hollow fiber.

The interaction component 18 thus directly affects the biological reaction. For example, at least one substrate, e.g. oxygen, carbon or nitrogen, and/or at least one inducer, e.g. specific sugars or enzymes, can be fed to the medium, on the one hand. On the other hand, at least one metabolite and/or at least one product can be withdrawn from the medium.

The structure of the interaction component 18 may also be adapted to draw at least one cell-free or cell-containing sample from the medium

In any event, the location(s) of the monitoring component(s) 16 and the interaction component(s) 18 within the fluid channel 10 are carefully chosen to examine and/or influence the biological reaction at given stages.

It is to be noted that the whole biomanufacturing assembly, including the fluid channel 12 as well as the monitoring component(s) 16 and the interaction component(s) 18, exclusively consists of single-use components.

According to the first embodiment of the disposable tubular biomanufacturing assembly shown in Figure 1, the fluid channel 10 is part of a stand-alone tubing. This means that basically no further components are necessary to stabilize the curved course of the fluid channel 10. To this end, the fluid channel 10 is either made of silicone or another suitable material, or it is reinforced to provide the required stability.

In the second embodiment shown in Figure 2, the fluid channel 10 is fixed in a cassette 20. As an option, further useful components or one or more further fluid channels 10 may be fixed therein. For facilitated access or other reasons, some or all of the components can be provided on one side of the cassette 20.

In the example according to Figure 2, the cassette 20 is divided into different temperature zones. Heating and/or cooling means are provided which can be controlled so that in a first portion 22 of the cassette 20 a defined first temperature is maintained and in a second portion 24 of the cassette 20 a defined second temperature is maintained. The locations of the monitoring component(s) 16 and the interaction component(s) 18 and the course of the fluid channel 10 are chosen such that the sections of the fluid channel 10 with the integrated components 16, 18 are located in a desired temperature zone, respectively.

Especially in a cassette design, the fluid channel 10 can have several individual output branches or openings at different locations with respect to the overall length of the fluid channel 10, so that the point of harvest can be selected as desired.

The fluid channel 10, or at least a section of it, may also be configured as a rigid piping as shown in Figure 3.

The embodiment of Figure 3 further features a mixing device 26, here in the form of baffles, integrated within the fluid channel 10. Further options for mixing the medium in the fluid channel 10 include integration of a static mixer and varying surface properties, especially surface friction, of the inner wall over the length of the fluid channel 10.

It is of course possible to combine certain features of the above-described embodiments in an appropriate manner.

Application fields which benefit from this invention include virus production, ultrafiltration, diafiltration and virus inactivation.

### List of Reference Signs

- 10: fluid channel
- 12: first end
- 14: second end
- 16: monitoring component
- 18: interaction component
- 20: cassette
- 22: first portion
- 24: second portion
- 26: mixing device

## Claims

1. A tubular biomanufacturing assembly consisting of single-use components, the biomanufacturing assembly comprising a continuous elongated fluid channel (10) for hosting a biological reaction occurring in a fluid medium along a flow path of the fluid channel (10), and at least one monitoring component (16) enabling monitoring of the biological reaction and/or at least one interaction component (18) for actively influencing the biological reaction, the monitoring component (16) and/or the interaction component (18) being integrated within the continuous elongated fluid channel (10).

2. The biomanufacturing assembly according to claim 1, **characterized in that** the monitoring component (16) includes at least one sensor, in particular one of the following: pH sensor; dissolved oxygen sensor; dissolved carbon dioxide sensor; microscope; fiber Bragg sensor; conductivity sensor; capacitance sensor; refractometer; flow sensor; pressure sensor; optical sensor; exhaust gas analyzer; image sensor.

3. The biomanufacturing assembly according to any of the preceding claims, **characterized in that** the interaction component (18) includes at least one structure adapted to exchange chemical substances with the medium in the fluid channel (10).

4. The biomanufacturing assembly according to claim 3, **characterized in that** the structure is adapted to feed at least one substrate and/or at least one inducer to the medium.

5. The biomanufacturing assembly according to claim 3 or 4, **characterized in that** the structure is adapted to withdraw dissolved carbon dioxide and/or at least one metabolite and/or at least one product from the medium.

6. The biomanufacturing assembly according to claim 5, **characterized in that** the structure is adapted to draw at least one sample from the medium.

7. The biomanufacturing assembly according to any of claims 3 to 6, **characterized in that** the structure includes a permeable or a semi-permeable membrane and/or a ceramic material.

8. The biomanufacturing assembly according to any of claims 3 to 6, **characterized in that** the structure includes at least one hollow fiber.

9. The biomanufacturing assembly according to any of the preceding claims, **characterized in that** the fluid channel (10) is part of a stand-alone tubing.

10. The biomanufacturing assembly according to any of claims 1 to 9, **characterized in that** the fluid channel (10) is part of a rigid piping.

11. The biomanufacturing assembly according to any of claims 1 to 9, **characterized in that** the fluid channel (10) is fixed in a cassette (20).

12. The biomanufacturing assembly according to any of the preceding claims, **characterized in that** at least one mixing device (26) is integrated within the fluid channel (10).

13. The biomanufacturing assembly according to any of the preceding claims, **characterized in that** different sections of the inner wall of the fluid channel (10) are made of materials with different surface properties.

14. The biomanufacturing assembly according to any of the preceding claims, **characterized in that** the course of the fluid channel (10) includes at least one meandering and/or at least one spiral section.

15. The biomanufacturing assembly according to any of the preceding claims, **characterized in that** different sections of the fluid channel (10) are heated or cooled differently.

16. The biomanufacturing assembly according to any of the preceding claims, **characterized in that**, the fluid channel (10) has several individual output and/or input branches and/or openings at different locations with respect to the overall length of the fluid channel (10).
